# EUROPEAN PATENT APPLICATION

(11) **EP 4 400 110 A1**
(43) Date of publication of application: **17.07.2024**
(21) Application number: 22864733.5
(22) Date of filing: 02.09.2022
(51) Int. Cl.: A61K 31/728, A61K 31/436, A61K 45/00, A61P 19/02, A61P 43/00, C12N 9/99

(54) **OSTEOARTHRITIS PROGRESSION INHIBITOR AND PROGRESSION INHIBITION KIT**

(30) Priority: 06.09.2021 JP 2021144702
(71) Applicant: Kyushu University, National University Corporation, Nishi-ku Fukuoka-shi Fukuoka 819-0395 (JP)
(72) Inventor: NAKASHIMA Yasuharu, Fukuoka-shi, Fukuoka 819-0395 (JP); AKASAKI Yukio, Fukuoka-shi, Fukuoka 819-0395 (JP); SUEISHI Takuya, Fukuoka-shi, Fukuoka 819-0395 (JP); TOYA Masakazu, Fukuoka-shi, Fukuoka 819-0395 (JP); KUWAHARA Masanari, Fukuoka-shi, Fukuoka 819-0395 (JP); UCHIDA Taisuke, Fukuoka-shi, Fukuoka 819-0395 (JP); TSUTSUI Tomoaki, Fukuoka-shi, Fukuoka 819-0395 (JP); TSUSHIMA Hidetoshi, Fukuoka-shi, Fukuoka 819-0395 (JP)
(74) Representative: EP&C
(86) International application number: PCT/JP2022/033150
(87) International publication number: WO 2023/033151

(57) **Abstract**

An osteoarthritis progression inhibitor contains, as active substances, a G protein-coupled receptor kinase 5 inhibitor, and hyaluronic acid or a pharmaceutically acceptable salt thereof. The G protein-coupled receptor kinase 5 inhibitor may be Amlexanox. The osteoarthritis progression inhibitor may be administered by intraarticular injection. An osteoarthritis progression inhibition kit contains a G protein-coupled receptor kinase 5 inhibitor, and hyaluronic acid or a pharmaceutically acceptable salt thereof.

## Description

### [Technical Field]

The present invention relates to an osteoarthritis progression inhibitor and a progression inhibition kit.

Priority is claimed on Japanese Patent Application No. 2021-144702, filed September 6, 2021, the content of which is incorporated herein by reference.

### [Background Art]

Osteoarthritis (OA) is a disease that causes cartilage to degenerate and wear out. Among others, knee osteoarthritis (hereinbelow, it may be abbreviated as "knee OA") is a typical disease that reduces the quality of life (QOL) of the elderly and threatens healthy life expectancy. As of 2010, the number of symptomatic patients with knee OA in Japan is estimated to be 8,000,000 (research on osteoarthritis against disability (ROAD) project).

Current medication therapy for OA is mainly symptomatic therapy to alleviate pain, and hyaluronic acid injection and steroid injection are commonly performed in clinical practice (for example, see Non-Patent Document 1).

The inventors have hitherto found that G protein-coupled receptor kinase 5 (GRK5) is highly expressed specifically in OA cartilage and aggravates cartilage degeneration through a catabolic response mediated by the NF-κB pathway, and the intraarticular administration of a GRK5 inhibitor may serve as a disease-modifying OA therapeutic agent capable of structurally inhibiting cartilage degeneration in OA (for example, see Non-Patent Document 2 and Patent Document 1).

Although a hyaluronic acid injection has been reported to have an anti-inflammatory effect, it is not a fundamental treatment method. Although a steroid injection has an excellent effect of inhibiting inflammation, the number of patients to which the steroid injection can be used is limited from the viewpoint of side effects. Thus, the steroid injection cannot be used frequently.

In addition, in the treatment methods using GRK5 inhibitors described in Patent Document 1 and Non-Patent Document 2, there is room for improvement in terms of reducing the frequency of use and improving the efficacy in order to reduce the burden on patients due to the injection.

### [Citation List]

### [Patent Document]

[Patent Document 1]
Japanese Unexamined Patent Application, First Publication No. 2020-121957

### [Non-Patent Documents]

[Non-Patent Document 1]
   Hiroshi KAWAGUCHI, "International and National Guidelines of Osteoarthritis Treatment", Japanese journal of joint diseases, 2016; 35(1): 1-9
[Non-Patent Document 2]
   Sueishi T et al., "GRK5 Inhibition Attenuates Cartilage Degradation via Decreased NF-κB Signaling.", Arthritis and Rheumatology, Vol. 72, No. 4, pp. 620-631, 2020.

### [Summary of Invention]

### [Technical Problem]

The present invention has been made in view of the above circumstances, and provides an osteoarthritis progression inhibitor and a progression inhibition kit capable of inhibiting the progression of OA and improving efficacy.

### [Solution to Problem]

As a result of intensive research to achieve the above purpose, the inventors found that simultaneous administration of hyaluronic acid and a GRK5 inhibitor can inhibit mRNA expressions of Interleukin 6 (IL6), which is a typical inflammatory cytokine involved in OA pathogenesis, Matrix metalloproteinase 13 (MMP13), which serves as a substrate-degrading enzyme, and a disintegrin and metalloproteinase with thrombospondin motifs 4 (ADAMTS4) more significantly than administration of the GRK5 inhibitor alone, thereby achieving the present invention.

That is, the present invention includes the following aspects.
(1) An osteoarthritis progression inhibitor, containing, as active substances:
   a G protein-coupled receptor kinase 5 inhibitor; and
   hyaluronic acid or a pharmaceutically acceptable salt thereof.
(2) The osteoarthritis progression inhibitor according to (1), in which the G protein-coupled receptor kinase 5 inhibitor is Amlexanox.
(3) The osteoarthritis progression inhibitor according to (1) or (2), in which the osteoarthritis progression inhibitor is administered by intraarticular injection.
(4) An osteoarthritis progression inhibition kit, containing:
   a G protein-coupled receptor kinase 5 inhibitor; and
   hyaluronic acid or a pharmaceutically acceptable salt thereof.

### [Advantageous Effects of Invention]

The OA progression inhibitor and progression inhibition kit of the above aspects can inhibit the progression of OA and improve efficacy.

### [Brief Description of Drawings]

FIG. 1 is a graph showing a relative expression level of each gene involved in the pathogenesis of OA in human OA cartilage cells in Example 1.
FIG. 2A is a diagram showing an administration schedule for an OA model mouse in Example 2.
FIG. 2B is safranin O-fast green stained images of knee joint tissue sections in each group in Example 2. The scale bar indicates 100 µm.
FIG. 2C is a graph quantifying the severity degree of OA in each group in Example 2.
FIG. 3A is a diagram showing an administration schedule for an OA model mouse in Example 3.
FIG. 3B is a safranin O-Fast Green stained image of a knee joint tissue section of a group in which Amlexanox and hyaluronic acid were administered every 10 days for 8 weeks in Example 3. The scale bar indicates 100 µm.
FIG. 3C is a graph quantifying the severity degree of OA in each group in Example 3.
FIG. 3D is a graph showing results of measuring joint pressure pain threshold in each group in Example 3.

### [Description of Embodiments]

### <<OA Progression Inhibitor>>

An OA progression inhibitor of the present embodiment contains, as active substances, a GRK5 inhibitor, and hyaluronic acid or a pharmaceutically acceptable salt thereof.

In addition, in the present specification, "contains something as an active substance" means to contain a therapeutically effective dose of a GRK5 inhibitor and either hyaluronic acid or a pharmaceutically acceptable salt thereof.

As shown in Examples described later, the inventors found a synergistic effect of inhibiting the gene expressions of II,6, which is an inflammatory cytokine, MMP13, which is a proteolytic enzyme, and ADAMTS4 by mixing Amlexanox serving as a GRK5 inhibitor with hyaluronic acid and allowing the mixture act on human cartilage cells. Furthermore, the inventors found a synergistic effect of structurally inhibiting cartilage degeneration in OAby intraarticularly administering the mixture of Amlexanox and hyaluronic acid to animal models. It was inferred that this is because the absorption of Amlexanox from the joint after the intraarticular administration can be slowed down by the simultaneous administration of Amlexanox and hyaluronic acid.

Osteoarthritis (OA) may occur in any joint of the body. In particular, sites with a high possibility of causing a deterioration in QOL due to the onset of OA and threatening healthy life expectancy are joints that play a role in supporting the body and have a large body weight load, specifically such as knee joints, hip joints, the spine, which are referred to as knee osteoarthritis (knee OA), hip osteoarthritis, and spinal osteoarthritis, respectively. Among these, the prophylactic or therapeutic agent for OA of the present embodiment is particularly suitably used for knee osteoarthritis.

In addition, OAis classified into primary arthritis and secondary arthritis depending on the cause thereof. The primary arthritis is considered to be OA that has occurred due to factors such as obesity and aging, although the cause thereof has not been specified. Most knee osteoarthritis is primary arthritis. On the other hand, the secondary arthritis is OAthat has developed due to a disease, injury, or the like. Specific examples of the cause of the onset include rheumatoid arthritis, gout, fracture, damage of ligaments and meniscus, congenital abnormal joint structure, and the like. Most hip osteoarthritis is secondary arthritis. Among these, the prophylactic or therapeutic agent for OA of the present embodiment is particularly suitably used for the primary arthritis.

### <GRK5 Inhibitor>

Exemplary examples of the GRK5 inhibitor include those that inhibit IκBα phosphorylation by GRK5. Specific examples of the GRK5 inhibitor include low-molecular-weight compounds, GRK5 expression inhibitors, GRK5-specific binding substances, and the like.

### [Low-Molecular-Weight Compound]

Exemplary examples of a low-molecular-weight compound serving as the GRK5 inhibitor include a compound represented by General Formula (I) (hereinafter, referred to as a "compound (I)" in some cases). The compound (I) is an example of the GRK5 inhibitor, although the GRK5 inhibitor is not limited thereto.

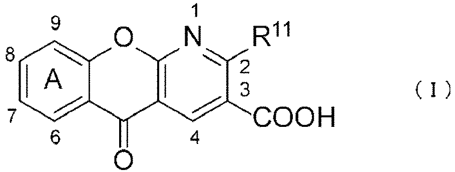

(In General Formula (I), a ring A may be substituted. R¹¹ is a hydrogen atom, an alkyl group, a phenyl group, an amino group, a carboxy group, or a hydroxyl group.)

### (Compound (I))

The compound (I) is a 1-azaxanthone-3-carboxylic acid derivative. The compound (I) will be described in detail below.

### · Ring A

A benzene ring represented by the ring A may be substituted. Exemplary examples of a substituent of the ring A include a halogen atom, a nitro group, an amino group, a mono- or dialkylamino group, an alkyl group, an alkoxy group, a carboxy group, and a hydroxyl group. In addition, the substituent may be a butadienylene group (-CH=CH-CH=CH-) in which two adjacent substituents consisting of carbon atoms among the substituents at the 6, 7, 8, or 9 position in the ring A form a benzene ring.

The alkyl group may be chain-like or cyclic. The chain-like alkyl group may be linear or may be branched. The chain-like alkyl group preferably has 1 or more and 6 or less carbon atoms, and specific examples thereof include a methyl group, an ethyl group, a propyl group, an isopropyl group, a butyl group, an isobutyl group, a t-butyl group, a pentyl group, hexyl group, and the like. Among these, a lower alkyl group having 1 or more and 3 or less carbon atoms is preferable in terms of practical use. Exemplary examples of the cyclic alkyl group include a cyclopentyl group, a cyclohexyl group, and the like.

Exemplary examples of the halogen atom include a chlorine atom, a bromine atom, an iodine atom, and a fluorine atom.

Exemplary examples of the mono- or dialkylamino group include amino groups substituted with lower alkyl groups having 1 or more and 3 or less carbon atoms, such as a methylamino group, an ethylamino group, a propylamino group, a dimethylamino group, a diethylamino group, and a dipropylamino group.

Exemplary examples of the alkoxy group include an alkoxy group, which is an alkyl group of which the alkyl moiety has 1 or more and 4 or less carbon atoms, such as a methoxy group, an ethoxy group, a propoxy group, or a butoxy group.

The ring A may have one or more of these substituents, but preferably has one of these substituents. In a case where the ring A has two or more substituents, the substituents may be the same as or different from each other. In addition, these substituents may be substituted at any positions on the ring A; however, out of those positions, a substituent is preferably substituted at the 7-position of the ring A.

### · R¹¹

R¹¹ is a hydrogen atom, an alkyl group, a phenyl group, an amino group, a carboxy group, or a hydroxyl group. The alkyl group is preferably a linear alkyl group having 1 or more and 6 or less carbon atoms, and specific examples thereof include a methyl group, an ethyl group, an n-propyl group, an n-butyl group, an n-hexyl group, and the like.

Among these, R¹¹ is preferably an amino group.

A specific example of the preferred compound (I) includes a compound represented by General Formula (I-1) (hereinafter, referred to as a "compound (I-1)" in some cases). The compound (I-1) is an example of the preferred compound (I), although the preferred compound (I) is not limited thereto.

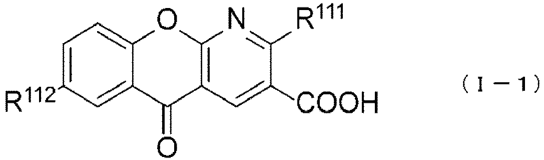

(In General Formula (I-1), R¹¹¹ is the same as R¹¹. R¹¹² represents a halogen atom, a nitro group, an amino group, a mono- or dialkylamino group, an alkyl group, an alkoxy group, a carboxy group, or a hydroxyl group.)

### · R¹¹¹

R¹¹¹ is the same as R¹¹ described above. Among these, R¹¹¹ is preferably an amino group.

### · R¹¹²

R¹¹² represents a halogen atom, a nitro group, an amino group, a mono- or dialkylamino group, an alkyl group, an alkoxy group, a carboxy group, or a hydroxyl group. Exemplary examples of the halogen atom, mono- or dialkylamino group, alkyl group, and alkoxy group include the same exemplary examples as those exemplified as the substituents of the ring A. Among these, R¹¹² is preferably an alkyl group, more preferably a chain-like alkyl group having 1 or more and 3 or less carbon atoms, and still more preferably an isopropyl group.

Exemplary examples of the preferred compound (I-1) include a compound with R¹¹¹ that is an amino group and R¹¹² that is an alkyl group.

Exemplary examples of the preferred compound (I-1) include a compound with R¹¹¹ that is an amino group and R¹¹² that is a chain-like alkyl group having 1 or more and 3 or less carbon atoms.

A specific example of the preferred compound (I-1) includes a compound represented by Formula (I-1-1) (hereinafter, referred to as a "compound (I-1-1)" in some cases). The compound (I-1-1) is an example of the preferred compound (I-1), although the preferred compound (I-1) is not limited thereto.

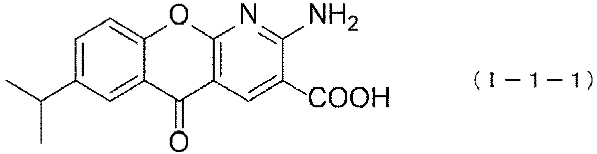

The compound (I-1-1) is 2-amino-7-isopropyl-5-oxo-5H-[1]benzopyrano[2,3-b]-pyridine-3-carboxylic acid, and its generic name is Amlexanox. It is known that Amlexanox exhibits a strong inhibitory effect on the histamine release reaction from IgE-involved mast cells and has strong anti-allergic and anti-inflammatory effects due to its inhibitory effect on leukotriene synthesis from macrophages (SRS-A production inhibitory effect) and antagonistic effect on leukotrienes (SRS-A antagonistic effect) (Reference 1: see "Japanese Unexamined Patent Application, First Publication No. S53-111096"). In addition, Amlexanox is clinically used as an anti-allergic agent in the form of nasal drops, eyedrops, and tablets under the trade name "SOLFA (registered trademark)" or the trade name "Elixir (registered trademark)". In recent years, it has been reported that Amlexanox selectively inhibits GRK5 (Reference 2: "Homan KT et al., "Identification and Characterization of Amlexanox as a GProtein-Coupled Receptor kinase 5 Inhibitor.", Molecules, Vol. 19, p16937-16949, 2014."). Since Amlexanox has been used clinically so far, Amlexanox is considered to be highly safe and is suitably used as the GRK5 inhibitor in the prophylactic or therapeutic agent for OA of the present embodiment.

In addition, exemplary examples of the salt of the compound (I) include an inorganic acid salt or an organic acid salt, and salts that are suitable for separation or crystallization of the compound (I). Specific examples of the salt of the compound (I) include an organic amine salt, an alkali metal salt, an ammonium salt, and a pharmaceutically acceptable salt.

Exemplary examples of the pharmaceutically acceptable salt of the compound (I) include, for example, a hydrochloride salt, a bromine salt, a sulfate salt, a hydrogen sulfate salt, a dihydrogen phosphate salt, a methanesulfonate salt, a methylsulfate salt, a maleate salt, a fumarate salt, a 2-naphthalene sulfonate salt, a benzenesulfonate salt, a glycolate salt, a gluconate salt, a citrate salt, an isethionate salt, a para-toluenesulfonate salt, and the like.

### (Method of Producing Compound (I))

The compound (I) can be produced, for example, using a method described below.

In other words, a compound represented by General Formula (Ia) (hereinafter, referred to as a "compound (Ia)" in some cases) is reacted with an active methylene compound and hydrolyzed to obtain the compound (I) (see Reference 2 described above).

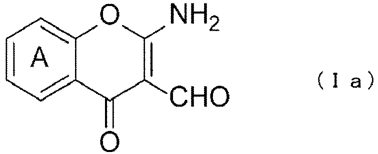

(In General Formula (Ia), a ring A may be substituted.)

Exemplary examples of the active methylene compound used in the reaction include methyl acetoacetate, ethyl acetoacetate, methyl cyanate, methyl cyanate, cyanoacetamide, malonitrile, oxaloacetate ethyl ester, diethyl malonate, dimethyl malonate, and benzoyl ethyl acetate, methyl-3-oxo-n-caproate, and the like. The amount of active methylene compound used is usually about 1 mol or more and 10 mol or less with respect to 1 mol of the compound (Ia).

Generally, the above-described reaction is desirably performed in the presence of a base, and exemplary examples of the base to be used include organic amines. Specific examples of the organic amines include a primary amine, a secondary amine, a tertiary amine, a heterocyclic base, and the like. Exemplary examples of the primary amine include n-butylamine, benzylamine, aniline, and the like. Exemplary examples of the secondary amine include diethylamine, dipropylamine, dibutylamine, piperidine, pyrrolidine, morpholine, and the like. Exemplary examples of the tertiary amine include 1,8-diazabicyclo[5,4,0]-7-undecene, triethylamine, and the like. Exemplary examples of the heterocyclic base include imidazole, 2-methylimidazole, and the like. The amount of base used is usually a catalyst amount or more and 5 mol or less with respect to 1 mol of the compound (Ia).

The above-described reaction is generally preferably performed in an organic solvent, and exemplary examples of the solvent to be used include alcohols, aromatic hydrocarbons, dimethylformamide, and the like. Exemplary examples of the alcohols include methanol, ethanol, propanol, butanol, and the like. Exemplary examples of the aromatic hydrocarbons include benzene, toluene, and the like.

The other reaction conditions such as a reaction temperature and a reaction time are not particularly limited, but the reaction is generally carried out at room temperature (about 25°C) or higher and a temperature near the boiling point of the solvent used or lower for about 1 hour or longer and 24 hours or shorter.

The compound obtained as described above is hydrolyzed to obtain the compound (I). As the condition for hydrolysis, the usual acidic hydrolysis method is employed. For example, the hydrolysis is carried out by using an excess of sulfuric acid, hydrochloric acid, phosphoric acid, or the like and heating only the acid or together with an organic solvent at a temperature of usually 50°C or higher and 150°C or lower. Exemplary examples of the organic solvent used for the hydrolysis include organic acids, alcohols, and the like. Exemplary examples of the organic acids include formic acid, acetic acid, and the like. Exemplary examples of the alcohols include methanol, ethanol, propanol, and the like. Although the reaction time varies depending on the kind of the desired compound (I), the reaction time is usually about 1 hour or longer and several days or shorter.

In addition, the compound (I) can also be produced, for example, using a method described below.

In other words, the compound (Ia) can be reacted with acetylene carboxylic acids, and the reaction can be hydrolyzed to obtain the compound (I).

Exemplary examples of the acetylene carboxylic acids used in the reaction include acetylene dicarboxylic acid dimethyl ester, acetylene dicarboxylic acid diethyl ester, ethyl propiolate, and the like. In a case where ethyl propiolate is used, an intermediate aminoacrylate derivative can be isolated, but the ring-closing reaction can be performed without isolation. The amount of acetylene carboxylic acid used is usually about 1 mol or more and 10 mol or less with respect to 1 mol of the compound (Ia).

It is generally desirable that the above-described reaction be performed in the presence of a base. Exemplary examples of the base to be used include organic amines. Exemplary examples of the organic amines include a secondary amine, a tertiary amine, a heterocyclic base, and the like. Exemplary examples of the secondary amine include piperidine, pyrrolidine, morpholine, diethylamine, dipropylamine, dibutylamine, and the like. Exemplary examples of the tertiary amine include triethylamine, tripropylamine, tributylamine, and the like. Exemplary examples of the heterocyclic base include pyridine, quinoline, imidazole, 2-methylimidazole, and the like. The amount of base used is usually a catalyst amount or more and 10 mol or less with respect to 1 mol of the compound (Ia).

The above-described reaction is generally preferably performed in an organic solvent, and exemplary examples of the solvent to be used include alcohols, aromatic hydrocarbons, dimethylformamide, and the like. Exemplary examples of the alcohols include methanol, ethanol, propanol, butanol, and the like. Exemplary examples of the aromatic hydrocarbons include benzene, toluene, xylene, and the like.

The other reaction conditions such as a reaction temperature and a reaction time are not particularly limited, but the reaction is generally carried out at room temperature or higher and a temperature near the boiling point of the solvent used or lower for about 1 hour or longer and 24 hours or shorter.

The compound obtained as described above is hydrolyzed to obtain the compound (I). As the conditions for the hydrolysis, the conditions described above can be used.

In addition, the compound (I) with R¹¹ that is a carboxy group is heated without a solvent at a temperature slightly higher than the temperature at which the decarboxylation reaction occurs to obtain the compound (I) with R¹¹ that is a hydrogen atom.

In addition, the compound (I) can be reacted with organic amines, an alkali metal hydroxide, ammonia, or the like by a known method such as mixing and heating the compound (I) in an appropriate solvent to obtain an organic amine salt, alkali metal salt, or ammonium salt of the compound (I).

### [GRK5 Expression Inhibitor]

Exemplary examples of a GRK5 expression inhibitor include siRNA, shRNA, miRNA, ribozyme, antisense nucleic acid, low-molecular-weight compounds, and the like. The administration of these GRK5 expression inhibitors enables the reduction of the expression level of GRK5 to inhibit a NF-κB signal. As a result, the expressions of inflammatory cytokines and substrate enzymes can be inhibited, thereby inhibiting the progression of OA. In other words, it is possible to prevent or treat OA by the administration of these GRK5 expression inhibitors.

A small interfering RNA (siRNA) is a small double-stranded RNA of 21 base pairs or more and 23 base pairs or less used for gene silencing by RNA interference. The siRNA introduced into the cell binds to RNA-induced silencing complex (RISC). This complex binds to and cleaves mRNA with a sequence complementary to siRNA. As a result, gene expression is inhibited in a sequence-specific manner.

The siRNA can be prepared by synthesizing sense strand and antisense strand oligonucleotides using a DNA/RNA automatic synthesizer individually, and for example, denaturing in an appropriate annealing buffer at about 90°C or higher and 95°C or lower for about 1 minute, and then annealing at about 30°C or higher and 70°C or lower for about 1 hour or more and 8 hours or less.

SiRNAs, shRNAs, miRNAs, ribozymes and antisense nucleic acids may contain various chemical modifications to improve stability and activity. For example, in order to prevent degradation by a hydrolase such as a nuclease, a phosphate residue may be replaced with a chemically modified phosphate residue such as phosphorothioate (PS), methylphosphonate, or phosphorodithionate. Furthermore, at least a portion thereof may be composed of a nucleic acid analogue, such as peptide nucleic acid (PNA).

### [GRK5-specific binding substance]

Exemplary examples of a GRK5-specific binding substance include those that specifically bind to GRK5 and inhibit the function of GRK5, and for example, include antibodies, antibody fragments, and aptamers. The antibody can be produced, for example, by immunizing an animal such as a mouse with the GRK5 protein or a fragment thereof as an antigen. Alternatively, the antibody can be prepared, for example, by screening a phage library. Exemplary examples of the antibody fragment include Fv, Fab, and scFv. The above antibody is preferably a monoclonal antibody. A commercially available antibody may also be used.

Aptamers are substances having a specific binding ability with respect to a target substance. Exemplary examples of aptamers include nucleic acid aptamers, peptide aptamers, and the like. Nucleic acid aptamers having a specific binding ability to a target peptide can be selected by, for example, a systematic evolution of ligand by exponential enrichment (SELEX) method or the like. The peptide aptamer having a specific binding ability to the target peptide can also be selected by, for example, a Two-hybrid method using yeast.

### <Hyaluronic acid or Pharmaceutically Acceptable Salt thereof>

Hyaluronic acid (hyaluronan) is basically a disaccharide or higher sugar containing at least a single disaccharide unit composed of 1-position of β-D-glucuronic acid and 3-position of β-D-N-acetylglucosamine, which are bonded to each other, and provided that a disaccharide unit is basically composed of β-D-glucuronic acid and β-D-N-acetylglucosamine, hyaluronan may be a sugar in which these elements bind to one or more disaccharide units binding to each other, or derivatives thereof, for example those with hydrolyzable protecting groups such as acyl groups, and the like. The sugar may be an unsaturated sugar, and exemplary examples of the unsaturated sugar include nonreducing terminal sugars, and usually, sugars in which the carbon atoms between 4- and 5-positions of glucuronic acid are unsaturated, and the like.

Specifically, as the hyaluronic acid, it is possible to use any of hyaluronic acids extracted from natural products such as animals, those obtained by culturing microorganisms, those chemically or enzymatically synthesized, and the like. For example, the hyaluronic acid can be obtained from a biological tissue, such as a crest, umbilical cord, skin, and joint fluid by an extraction method and a purification method known in the art. In addition, it can also be produced by a fermentative method using streptococcal bacteria and the like.

Hyaluronan oligosaccharide is also included in hyaluronic acid, and hyaluronic acids from a low-molecular-weight hyaluronic acid such as the disaccharide consisting of a single disaccharide unit described above and a derivative thereof to a high-molecular-weight hyaluronic acid whose weight-average molecular weight is as high as about 4,000,000 can be employed. From the viewpoint of excellent permeability in a tissue and the like, a hyaluronic acid whose weight-average molecular weight is about 380 or more and 4000,000 or less is preferred, and hyaluronic acid composed of disaccharide or higher sugar and 20 saccharides or lower sugar is more preferred.

It is possible to produce hyaluronic acid having a low-molecular-weight specifically by reducing the molecular weight of the hyaluronic acid using a known method such as an enzymatic degradation, an alkaline degradation, a heat treatment, and an ultrasonication, or by synthesizing the hyaluronic acid having a low-molecular-weight chemically or enzymatically. Exemplary examples of the enzymatic degradation include a method in which an enzyme capable of degrading the hyaluronic acid such as hyaluronan degradation enzyme (hyaluronidase (derived from testes), hyaluronidase (derived from Streptomyces), hyaluronidase SD and the like), chondroitinase AC, chondroitinase ACII, chondroitinase ACIII, and chondroitinase ABC is allowed to act on the hyaluronan to yield hyaluronan oligosaccharide, and the like.

In addition, exemplary examples of the alkaline degradation include a method of adding a base such as about 1N sodium hydroxide to a solution of hyaluronic acid which is then warmed for several hours to reduce the molecular weight, and then adding an acid such as hydrochloric acid for neutralization to obtain a low-molecular-weight hyaluronic acid, and the like.

Hyaluronic acid includes its salt form, and a pharmaceutically acceptable salt can be employed depending on formulation requirements. Exemplary examples thereof include alkali metal salts such as sodium salts and potassium salts, alkaline earth metal salts such as calcium salts and magnesium salts, amine salts such as tri(n-butyl)amine salts, triethylamine salts, pyridine salts, amino acid salts, and the like.

Exemplary examples of commercially available hyaluronic acids that are used clinically include ARTZ Dispo (purified sodium hyaluronate, a weight-average molecular weight of 500,000 or more and 1,200,000 or less), SUVENYL (purified sodium hyaluronate, a weight-average molecular weight of 1,500,000 or more and 3,900,000 million or less), and the like.

The GRK5 inhibitor described above, and the hyaluronic acid or a pharmaceutically acceptable salt thereof described above may be in the form of a mixture thereof, or the GRK5 inhibitor described above, and the hyaluronic acid or a pharmaceutically acceptable salt thereof described above may be separately sealed in a container and may be in the form of a kit that is adjusted at the time of use. In other words, in one embodiment, the present invention provides an osteoarthritis progression inhibition kit containing the GRK5 inhibitor, and the hyaluronic acid or a pharmaceutically acceptable salt thereof.

### <Pharmaceutical Composition>

The OA progression inhibitor of the present embodiment can be combined with a pharmaceutically acceptable carrier to form a pharmaceutical composition for inhibiting the progression of OA.

According to the pharmaceutical composition of the present embodiment, the progression of OA can be inhibited, and the efficacy is improved as compared with the administration of the GRK5 inhibitor alone.

The pharmaceutical composition of the present embodiment may have a dosage form to be used orally or may have a dosage form to be used parenterally, and the dosage form to be used parenterally is preferable. Exemplary examples of the dosage form to be used orally include tablets, capsules, elixirs, microcapsules, and the like. Exemplary examples of the dosage form to be used parenterally include an injection agent, an ointment, an adhesive skin patch, and the like.

As the pharmaceutically acceptable carrier, those that are used for the formulation of a common pharmaceutical composition can be used without particular limitation. More specific examples thereof include binders such as gelatin, cornstarch, gum tragacanth, and gum arabic; excipients such as starch and crystalline cellulose; swelling agents such as alginic acid; solvents for an injection agent such as water, ethanol, and glycerin; adhesives such as a rubber-based adhesive and a silicone-based adhesive, and the like.

The pharmaceutical composition may contain an additive. Exemplary examples of the additive include lubricants such as calcium stearate and magnesium stearate; sweetening agents such as sucrose, lactose, saccharin, and maltitol; flavoring agents such as peppermint and "Akamono" (Gaultheria adenothrix) oil; stabilizers such as benzyl alcohol and phenol; buffers such as a phosphoric acid salt and sodium acetate; solubilization agents such as benzyl benzoate and benzyl alcohol; antioxidants; preservatives, and the like.

The pharmaceutical composition can be formulated by appropriately combining and mixing the above-described OA progression inhibitor with the above-described pharmaceutically acceptable carrier and additive in a unit dose form required for generally accepted pharmaceutical practice.

The pharmaceutical composition may be used in combination with at least one selected from the group consisting of a therapeutic agent with the anti-inflammatory effect other than the above-described OA progression inhibitor, and a therapeutic agent for another disease. The above-described OA progression inhibitor and the other agent may be in the same formulation or may be in separate formulations. In addition, each formulation may be administered by the same administration route, or may be administered by separate administration routes. Furthermore, each formulation may be administered simultaneously, may be administered sequentially, or may be administered separately after a certain time or period. In one embodiment, the OA progression inhibitor and the other agent may be included in a kit.

### <Administration Method>

An administration subject includes, but is not limited to, humans, monkeys, dogs, cows, horses, sheep, pigs, rabbits, mice, rats, guinea pigs, hamsters, and cells and the like thereof. Among these, mammals or mammalian cells are preferable, and humans or human cells are particularly preferable.

The administration route is preferably a parenteral administration route such as subcutaneous administration, transdermal administration, intramuscular administration, and intraarticular administration, and particularly preferably intraarticular administration.

The dosage of the OA progression inhibitor varies depending on the types of the GRK5 inhibitor and the hyaluronic acid or a pharmaceutically acceptable salt thereof, symptoms of administration subjects, administration sites, administration methods, and the like. The appropriate selection of a suitable dosage is possible by those skilled in the art, and for example, in the case of topical administration, the dosage of the GRK5 inhibitor is generally about 0.001 mg or more and about 10 mg or less, preferably about 0.01 mg or more and about 5 mg or less, and more preferably about 0.02 mg or more and about 2 mg or less per day for adults (assuming a body weight of 60 kg). The dosage of hyaluronic acid or a pharmaceutically acceptable salt thereof is generally about 0.08 mg or more and about 800 mg or less, preferably about 0.8 mg or more and about 400 mg or less, and more preferably about 1.6 mg or more and about 100 mg or less per day for adults (assuming a body weight of 60 kg) in the usual case. Alternatively, since one syringe (2.5 mL) (containing about 25 mg as purified sodium hyaluronate) is usually administered in clinical practice, hyaluronic acid or a pharmaceutically acceptable salt thereof can be administered within a range of 25 mg or more or 25 mg or less per dose.

The administration of the OA progression inhibitor may be a single dose, or may be multiple doses. In the case of the multiple doses, for example, the administration can be carried out once every period of 2 hours or longer and 12 hours or shorter, every day, or every 2 days, 5 days, 1 week, 1.5 weeks, several weeks, one month, several months or the like. Usually, in clinical practice, for the treatment of OA, one syringe (25 mg once as purified sodium hyaluronate) for adults is administered into the articular cavity of the knee joint five times consecutively every week, and then, in a case where the purpose is to maintain symptoms, the administration is carried out at intervals of two weeks or more and four weeks or less. Meanwhile, as shown in Examples below, the OA progression inhibitor of the present embodiment is used in combination with the GRK5 inhibitor and the hyaluronic acid or a pharmaceutically acceptable salt thereof, thereby obtaining a sufficient effect even in a case where the administration is carried out at intervals, which enables the reduction of the number of administrations compared to the conventional methods.

### <<Other Embodiments>>

In one embodiment, the present invention provides a method of preventing, treating, or inhibiting the progression of OA including administering effective doses of a GRK5 inhibitor and hyaluronic acid or a pharmaceutically acceptable salt thereof to a human patient or an animal patient in need of such treatment. Here, exemplary examples of the GRK5 inhibitor and the hyaluronic acid or a pharmaceutically acceptable salt thereof are the same as those described above. In addition, as the type of OA, the same ones as described above are exemplary examples.

In one embodiment, the present invention provides a GRK5 inhibitor and hyaluronic acid or a pharmaceutically acceptable salt thereof for preventing, treating, or inhibiting the progression of OA. Here, exemplary examples of the GRK5 inhibitor and the hyaluronic acid or a pharmaceutically acceptable salt thereof are the same as those described above. In addition, as the type of OA, the same ones as described above are exemplary examples.

In one embodiment, the present invention provides the use of a GRK5 inhibitor, and hyaluronic acid or a pharmaceutically acceptable salt thereof in order to produce an OA progression inhibitor. Here, exemplary examples of the GRK5 inhibitor and the hyaluronic acid or a pharmaceutically acceptable salt thereof are the same as those described above. In addition, as the type of OA, the same ones as described above are exemplary examples.

### [Examples]

Hereinafter, the present invention will be described with reference to Examples, but the present invention is not limited to the following Examples.

### [Materials]

### 1. Collection of Human Cartilage Cells

Human cartilage cells were isolated and cultured from an excised cartilage fragment that is usually discarded after artificial knee joint replacement surgery. All donors provided consent to participate in the clinical research approved by the Ethics Review Committee. Due consideration was given to personal information of all donors in handling. Donor's knee joints were OA grade (Kellgren-lawrence) III to IV on X-rays.

### 2. Mice

C57BL/6J mice (hereinafter, may be referred to as "wild-type mice") were purchased from Charles River Japan. GRK5 knockout (KO) mice (GRK5-/-) were obtained from Dr. Kurose (Faculty of Pharmaceutical Sciences, Kyushu University). In wild-type mice and GRK5KO mice, there were no significant differences in growth plate thickness and cartilage thickness throughout joint surfaces, weight, appearance, and the like, and no differences in development. Immature cartilage cells were collected from 5-day-old mouse knees.

### [Example 1]

### (Synergistic Inhibitory Effect on Expressions of IL6, MMP13, and ADAMTS4 in Human Cartilage Cells by Simultaneous Administration of Amlexanox and Hyaluronic Acid)

Amlexanox (concentration in medium: 100 µM) and hyaluronic acid (concentration in medium: 1 mg/mL, weight-average molecular weight: 500,000 or more and 1,490,000 or less) were added to human OA cartilage cells and incubated for 48 hours. As a control, those that had been incubated for 48 hours without any administration, and those that were administered with Amlexanox (concentration in medium: 100 µM) and hyaluronic acid (concentration in medium: 1 mg/mL, weight-average molecular weight: 500,000 or more and 1,490,000 or less) and incubated for 48 hours were also prepared.

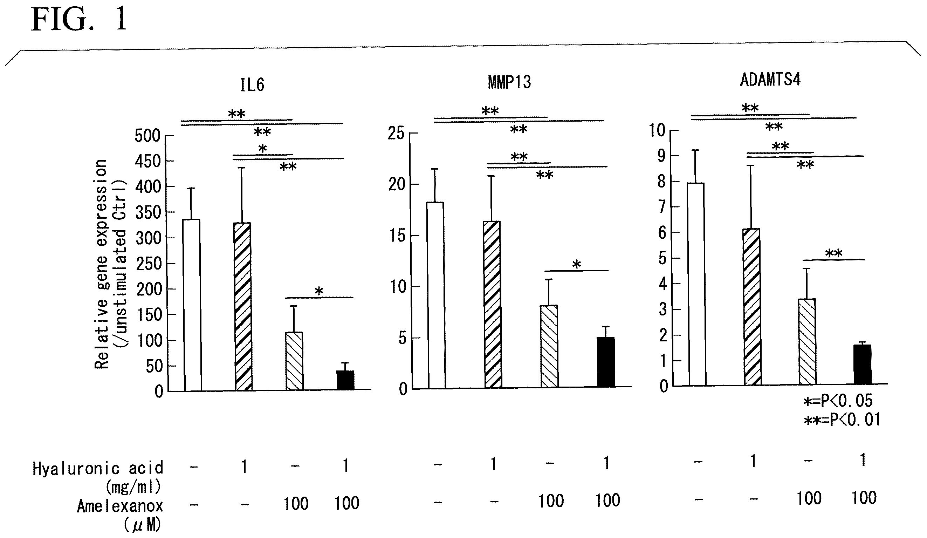

Human OA cartilage cells into which Amlexanox and hyaluronic acid were added were stimulated with lipopolysaccharide (LPS) (concentration in medium: 10 µg/mL) and incubated for 6 hours. Thereafter, mRNA was recovered from the above cells. After reverse transcription, quantitative real-time PCR was performed using LightCycler 2.0 system (Roche). GAPDH was used as an internal standard. The sequences of the primers used are shown in Table 1 below. The results are shown in FIG. 1.

**[Table 1]**

| | | 5'→3' | SEQ ID NO. |
|---|---|---|---|
| IL-6 | Forward primer | GGTACATCCTCGACGGCATCT | 1 |
| | Reverse primer | GTGCCTCTTTGCTGCTTTCAC | 2 |
| MMP13 | Forward primer | GTCTCTCTATGGTCCAGGAGATGAA | 3 |
| | Reverse primer | AGGCGCCAGAAGAATCTGT | 4 |
| ADAMTS4 | Forward primer | AGGCACTGGGCTACTACTAT | 5 |
| | Reverse primer | GGGATAGTGACCACATTGTT | 6 |
| GAPDH | Forward primer | GGTGAAGGTCGGAGTCAACGGA | 7 |
| | Reverse primer | GAGGGATCTCGCTCCTGGAAGA | 8 |

As shown in FIG. 1, no significant decrease in the expressions of IL6, MMP13, and ADAMTS4 was observed with the administration of hyaluronic acid alone (control ratio: 96.6%, 90.13%, 76.84%). The expressions of all of IL6, MMP13, and ADAMTS4 were significantly decreased with the administration of Amlexanox alone (control ratio: 32.99%, 43.83%, 42.04%). The control ratio was 10.58%, 26.25%, 18.95% with the simultaneous administration of Amlexanox and hyaluronic acid, and an Amlexanox alone ratio was 32.07%, 59.89%, 45.08%, so that a significant inhibition in the expressions of IL6, MMP13, and ADAMTS4 was observed, which showed a synergistic effect.

### [Example 2]

### (Synergistic Inhibitory Effect 1 on Progression of Cartilage Degeneration of Osteoarthritis by Simultaneous Intraarticular Administration of Amlexanox and Hyaluronic Acid)

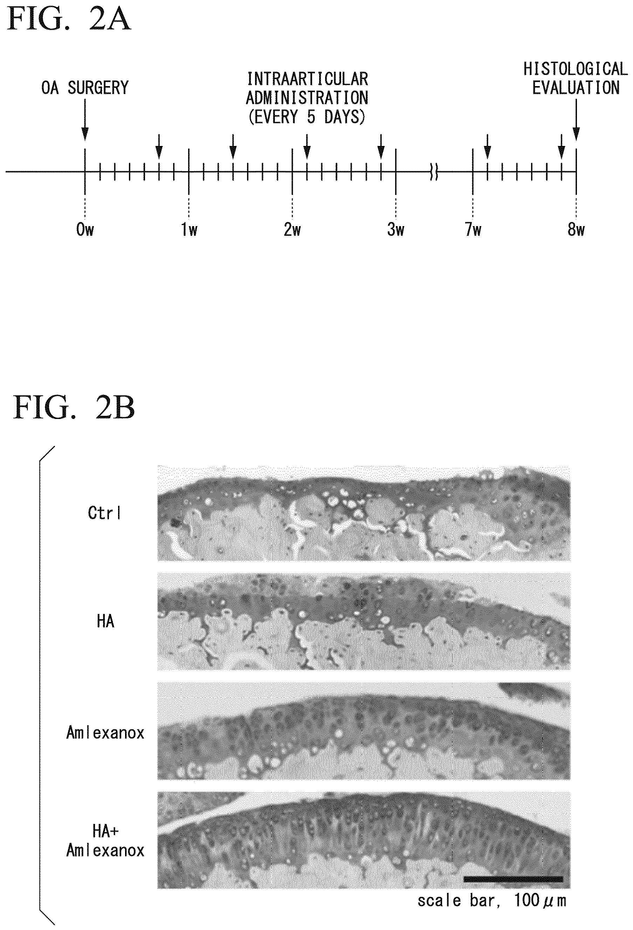

OA was induced in 12-week-old male mice (wild type mice and GRK5KO mouse) by removing the ligament, which connects and secures the medial meniscus to the tibia, to cause the knee joint instability (DMM model) (hereinafter, referred to as a "DMM group" in some cases). In addition, as a control, mice subjected to a sham operation by the same method without removing the ligament were also prepared (hereinafter, referred to as a "sham group" in some cases). Subsequently, immediately after the OA induction, four groups were classified as follows: a group administered with physiological saline (10 µL) every 5 days for 8 weeks; a group administered with a hyaluronic acid solution (10 mg/mL, 10 µL); a group administered with an Amlexanox solution (100 µM, 10 µL), a group administered with a mixed solution (10 µL) of Amlexanox (100 µM) and hyaluronic acid (10 mg/mL) (see FIG. 2A). Knee joint tissue sections of each group at a time point of 8 weeks after OA induction surgery were stained with safranin O. The results are shown in FIG. 2B (staining image).

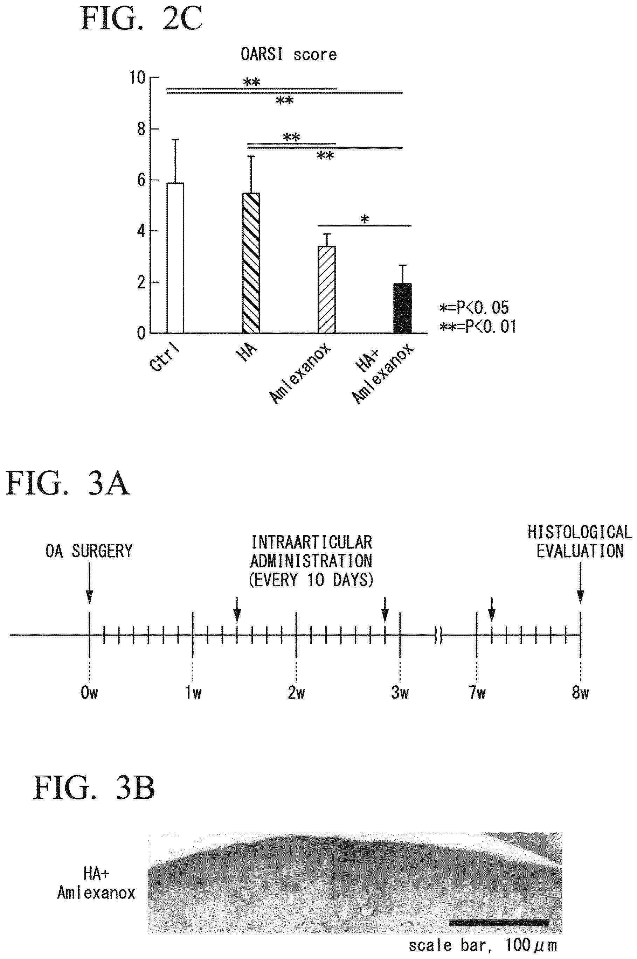

In addition, the severity degree of OA was quantified by evaluating the stained sections based on histopathological grade recommended by OARSI. The results are shown in FIG. 2C.

As shown in FIGS. 2B and 2C, there was no significant improvement in the OARSI score in the group administered with hyaluronic acid alone (control group ratio: 92.57%). In the group administered with Amlexanox alone, a significant decrease in the OARSI score was observed (control group ratio: 56.95%). In the group simultaneously administered with Amlexanox and hyaluronic acid, a control group ratio was 32.58%, and an Amlexanox alone group ratio was 57.21%, so that a significant decrease in the OARSI score was observed, which showed a synergistic effect of inhibiting cartilage degeneration.

It was inferred that this is because the absorption of Amlexanox from the joint after the intraarticular administration could be slowed down by the simultaneous administration of Amlexanox and hyaluronic acid. The half-life of hyaluronic acid in joint fluid is about 20 hours, and the hyaluronic acid is detected in the joint fluid until about 3 days after the administration. Amlexanox is a compound having a low-molecular-weight of 300 and has a half-life of several hours in the joint. It was conceived that a sustained drug efficacy could be obtained by the simultaneous administration of Amlexanox and hyaluronic acid, and was more effective in the mouse model than the administration of Amlexanox alone.

### [Example 3]

### (Synergistic Inhibitory Effect 2 on Progression of Cartilage Degeneration of Osteoarthritis by Simultaneous Intraarticular Administration of Amlexanox and Hyaluronic Acid)

Using the same method as in Example 2, a mixed solution of Amlexanox (100 µM) and hyaluronic acid (10 mg/mL) was administered to OA-induced mice every 10 days for 8 weeks (see FIG. 3A). Knee joint tissue sections of each group at a time point of 8 weeks after OA induction surgery were stained with safranin O. The results are shown in FIG. 3B (staining image).

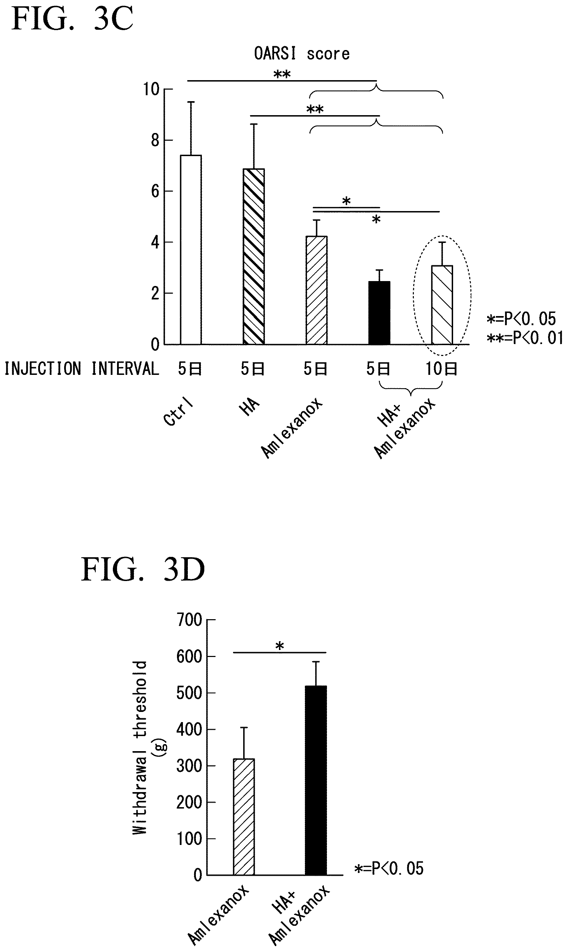

In addition, the severity degree of OA was quantified by evaluating the stained sections based on histopathological grade recommended by OARSI. The results are shown in FIG. 3C. FIG. 3C also shows the results of the four groups examined in Example 2.

As shown in FIGS. 3B and 3C, even though the administration interval was extended to 10 days, the effect of inhibiting cartilage degeneration was significantly higher in the group simultaneously administered with Amlexanox and hyaluronic acid than in the group administered with Amlexanox alone.

In addition, in the group of OA-induced mice administered with the mixed solution of Amlexanox (100 µM) and hyaluronic acid (10 mg/mL) every 10 days for 8 weeks, and the group of OA-induced mice administered with the Amlexanox solution (100 µM, 10 µL) every 5 days for 8 weeks, the joint pressure pain threshold was measured at 8 weeks after OA-induced surgery by using a SMALGO small animal algometer (manufactured by Bioseb, model number BIO-SMALGO+). The results are shown in FIG. 3D.

As shown in FIG. 3D, it was clarified that pain could be reduced in the group simultaneously administered with Amlexanox and hyaluronic acid as compared with the group administered with Amlexanox alone.

From the above results, the effect of inhibiting the progression of OA is synergistic due to the simultaneous administration of Amlexanox serving as the GRK5 inhibitor, and hyaluronic acid, and it is possible to obtain a disease-modifying OA therapeutic agent having an effect of inhibiting the progression of cartilage degeneration which does not yet exist. Since the administration of hyaluronic acid alone does not have an effect of inhibiting the progression of OA, the above-described results can be said to be a novel effect of hyaluronic acid capable of enhancing the effect of the GRK5 inhibitor (Amlexanox).

### [Industrial Applicability]

According to the osteoarthritis progression inhibitor and progression inhibition kit of the present embodiment, the progression of OA can be inhibited, and the efficacy is improved as compared with the administration of the GRK5 inhibitor alone.

## Claims

1. An osteoarthritis progression inhibitor, comprising, as active substances:
a G protein-coupled receptor kinase 5 inhibitor; and
hyaluronic acid or a pharmaceutically acceptable salt thereof.

2. The osteoarthritis progression inhibitor according to Claim 1, wherein the G protein-coupled receptor kinase 5 inhibitor is Amlexanox.

3. The osteoarthritis progression inhibitor according to Claim 1 or 2, wherein the osteoarthritis progression inhibitor is administered by intraarticular injection.

4. An osteoarthritis progression inhibition kit, comprising:
a G protein-coupled receptor kinase 5 inhibitor; and
hyaluronic acid or a pharmaceutically acceptable salt thereof.
